# EUROPÄISCHE PATENTSCHRIFT

(11) **EP 1 263 731 B1**
(45) Veröffentlichungstag und Bekanntmachung des Hinweises auf die Patenterteilung: **11.08.2010**
(21) Anmeldenummer: 01907409.5
(22) Anmeldetag: 05.01.2001
(51) Int. Cl.: C07D 213/48, C07D 213/80, C07D 213/30

(54) **VERFAHREN ZUR HERSTELLUNG VON 5-ARYLNICOTINALDEHYDEN**
METHOD FOR PRODUCING 5-ARYL NICOTINALDEHYDES
PROCEDE DE PREPARATION 5-ARYLNICOTINALDEHYDES

(30) Priorität: 07.02.2000 DE 10005150
(43) Veröffentlichungstag der Anmeldung: 11.12.2002
(73) Patentinhaber: Merck Patent GmbH, 64293 Darmstadt (DE)
(72) Erfinder: BATHE, Andreas, 64283 Darmstadt (DE); BOKEL, Heinz, 64287 Darmstadt (DE); KEIL, Thomas, 64354 Reinheim (DE); KNIERIEMEN, Ralf, 64846 Gross-Zimmern (DE); MÜRMANN, Christoph, 64354 Reinheim (DE)
(86) Internationale Anmeldenummer: PCT/EP2001/000083
(87) Internationale Veröffentlichungsnummer: WO 2001/058873

(56) Entgegenhaltungen:
- EP-A- 0 707 007
- EP-A- 0 908 458
- US-A- 5 585 388
- K NAGAYAMA ET AL: "Direct Hydrogenation of Carboxylic Acids to Corresponding Aldehydes Catalyzed by Palladium Complexes in the Presence of Pivalic Anhydride" CHEMISTRY LETTERS., Nr. 11, 1988, Seiten 1143-1144, XP000999904 CHEMICAL SOCIETY OF JAPAN. TOKYO., JP ISSN: 0366-7022 in der Anmeldung erwähnt
- MARQUAIS S ET AL: "Aryl-Aryl Cross Coupling on a Solid Support using Zinc Organic Reagents and Palladium Catalysis" TETRAHEDRON LETTERS,NL,ELSEVIER SCIENCE PUBLISHERS, AMSTERDAM, Bd. 37, Nr. 31, 29. Juli 1996 (1996-07-29), Seiten 5491-5494, XP004029432 ISSN: 0040-4039
- W J THOMSON ET AL: "An efficient synthesis of arylpyrazines in bipyridines" JOURNAL OF ORGANIC CHEMISTRY,US,AMERICAN CHEMICAL SOCIETY. EASTON, Bd. 53, 1988, Seiten 2052-2055, XP002143541 ISSN: 0022-3263
- LAPUYADE G ET AL: "DERIVES ALKYLES ET ARYLES DE L'ACIDE NIPECOTIQUE: SYNTHESE ET APPRECIATION DE L'ACTIVITE INHIBITRICE DE LA CAPTURE DU GABA EN FONCTION DE PARAMETRES CONFORMATIONNELS ET DE BIODISPONIBILITE. ALKYL AND ARYL DERIVATIVES OF NIPECOTIC ACID: SYNTHESIS AND INHIBITION OF GABA UPTAKE AS A FUNCTION OF CONFORM" EUROPEAN JOURNAL OF MEDICINAL CHEMISTRY.CHIMICA THERAPEUTICA,FR,EDITIONS SCIENTIFIQUE ELSEVIER, PARIS, Bd. 22, Nr. 5, 1. September 1987 (1987-09-01), Seiten 383-391, XP002035295 ISSN: 0223-5234
- W J THOMSON ET AL: JOURNAL OF ORGANIC CHEMISTRY., Bd. 49, Nr. 26, 1984, Seiten 5237-5243, XP000999537 AMERICAN CHEMICAL SOCIETY. EASTON., US ISSN: 0022-3263

## Beschreibung

Die Erfindung betrifft ein Verfahren zur Herstellung von 5-(4-Fluorphenyl)nicotinaldehyd, durch Reduktion der entsprechenden 5-Arylnicotinsäure mittels katalytischer Hydrierung in Gegenwart von Carbonsäureanhydriden, bei der als Katalysator ein Palladium-Ligand-Komplex verwendet wird, dadurch gekennzeichnet, daß das molare Verhältnis von Palladium zu Ligand bei einzähnigen Liganden bei 1 zu 5 bis 1 zu 15 und bei zweizähnigen Liganden bei 1 zu 2,5 bis 1 zu 7,5 liegt.

5-Arylnicotinaldehyde sind bedeutende Zwischen- oder Endprodukte in der industriellen Organischen Chemie. Entsprechend substituierte Derivate stellen insbesondere wertvolle Zwischenprodukte zur Synthese von hochveredelten Endprodukten dar oder sind selbst solche Endprodukte insbesondere für den Pflanzenschutz, wie z.B. Fungizide, Insektizide, Herbizide oder Pestizide oder zur Herstellung von pharmazeutisch hochwirksamen Substanzen. Eine Produktion der entsprechenden 5-Arylnicotinaldehyde im großtechnischen Maßstab macht die möglichst wirtschaftliche und umweltverträgliche Darstellung erforderlich.

5-Arylnicotinaldehyde zeichnen sich durch eine unerwarte Oxidations- und Disproportionierungsempfindlichkeit aus. Dadurch sind die in der Literatur beschriebenen Methoden zur Herstellung dieser Verbindungen durch chemoselektive Reduktion von aromatischen Säuren bzw. deren Ester oder durch Oxidation von aromatischen Alkoholen nicht in zufriedenstellendem Maß auf die entsprechende 5-Arylnicotinsäure bzw. den entsprechenden 5-Arylnicotinalkohol anwendbar.

Methoden zur Reduktion der Nicotinsäure sind bekannt. So wird beispielsweise die Reduktion von Nicotinsäure durch Palladiumkatalysierte Hydrierung in Chemistry Letters 1998, 1143-1144 beschrieben. In dem darin offenbarten Verfahren wird in Gegenwart von Pivalinsäureanhydrid (Trimethylessigsäureanhydrid) gearbeitet, wodurch bei der unsubstituierten Nicotinsäure der entsprechende Aldehyd in guten Ausbeuten erhalten wird.

Setzt man bei diesen Verfahren jedoch substituierte Nicotinsäuren und insbesondere durch Aromaten substituierte Nicotinsäuren ein, treten erhebliche Ausbeuteverluste ein. Zusätzlich ist die Verwendung von Pivalinsäure mit erheblichen Kosten verbunden, so daß dieses Verfahren für industrielle Anwendungen nicht in Frage kommt.

Die der Erfindung zugrundeliegende Aufgabe bestand darin, ein Verfahren zur Herstellung von 5-(4-Fluorphenyl)nicotinaldehyd aufzufinden, das einen kostengünstigen und umweltfreundlichen Zugang ermöglicht, ohne die oben genannten Nachteile aufzuweisen.

Es wurde nun überraschend gefunden, daß sich 5-(4-Fluorphenyl)nicotinaldehyd durch Reduktion der entsprechenden 5-Arylnicotinsäure mittels katalytischer Hydrierung in Gegenwart von Carbonsäureanhydriden, bei der als Katalysator ein Palladium-Ligand-Komplex verwendet wird, erhalten lassen, wenn ein molares Verhältnis von Palladium zu Ligand bei einzähnigen Liganden bei 1 zu 5 bis 1 zu 15 und bei zweizähnigen Liganden bei 1 zu 2,5 bis 1 zu 7,5 eingehalten wird.

Durch die erfindungsgemäße Maßnahme können auch andere, kostengünstigere Carbonsäureanhydride als Pivalinsäureanhydrid Verwendung finden, die ohne Ligandenüberschuss nur eine geringe Ausbeute an Produkt liefern.

Gegenstand der Erfindung ist somit ein Verfahren zur Herstellung von 5-(4-Fluorphenyl)nicotinaldehyd, durch Reduktion der entsprechenden 5-Arylnicotinsäure mittels katalytischer Hydrierung in Gegenwart von Carbonsäureanhydriden, bei der als Katalysator ein Palladium-Ligand-Komplex verwendet wird, dadurch gekennzeichnet, daß das molare Verhältnis von Palladium zu Ligand bei einzähnigen Liganden bei 1 zu 5 bis 1 zu 15 und bei zweizähnigen Liganden bei 1 zu 2,5 bis 1 zu 7,5 liegt. Weiterer Gegenstand der Erfindung ist die Verwendung von 5-(4-Fluorphenyl)nicotinaldehyd zur Herstellung von N-alklierten Aminen durch Umsetzung von 5-(4-Fluorphenyl)nicotinaldehyd mit primären Aminen zu den entsprechenden Iminen und nachfolgender Reduktion. Die Umsetzung des Aldehyds mit den Aminen und auch die Reduktion der Imine kann nach an sich bekannten Methoden durchgeführt werden, wie sie in der Literatur beschrieben sind (z.B. in Standardwerken wie Houben-Weyl, Methoden der organischen Chemie, Georg-Thieme-Verlag, Stuttgart oder Organikum, 17. Auflage, VEB Deutscher Verlag der Wissenschaften, Berlin 1988) und zwar unter Reaktionsbedingungen, die für die genannten Umsetzungen bekannt und geeignet sind.

Ähnliche Verbindungen sind bereits aus EP 0707007 bekannt.

Bevorzugt wird der Aldehyd der Formel I, worin worin A die Bedeutung 4-Fluorphenyl aufweist, durch Umsetzung mit R-Chromanamin und Reduktion des erhaltenen Imins durch übliche Reduktionsverfahren, insbesondere mittels katalytischer Hydrierung oder durch Anwendung von Borhydriden wie z. B. Natriumborhydrid, zur Herstellung von (R)-2-[5-(4-Fluorophenyl)-3-pyridylmethylaminomethyl]-chroman eingesetzt.

Die als Ausgangsstoffe für das erfindungsgemäße Verfahren verwendete 5-Arylnicotinsäure ist entweder bekannt oder wird nach an sich bekannten Methoden hergestellt, wie sie in der Literatur beschrieben sind (z.B. in Standardwerken wie Houben-Weyl, Methoden der organischen Chemie, Georg-Thieme-Verlag, Stuttgart) und zwar unter Reaktionsbedingungen, die für die genannten Umsetzungen bekannt und geeignet sind. Man kann aber auch von an sich bekannten, hier nicht näher erwähnten Varianten Gebrauch machen.

Vorzugsweise wird die 5-Arylnicotinsäure durch Suzuki-Kupplung (N. Miyaura, A. Suzuki, Chem. Rev. 95, 2457 (1995)) hergestellt, indem man 5-Brom-nicotinsäure, 5-Brom-nicotinsäurealkylester, 5-Brom-nicotinalkohol oder seinen Alkylcarbonsäureester mit den entsprechenden Arylboronsäuren unter bekannten Reaktionsbedingungen zu den Verbindungen der Formeln II, III oder IV oder V umsetzt: 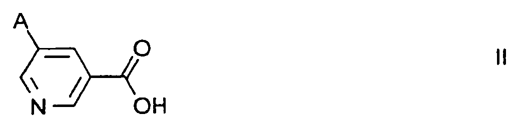 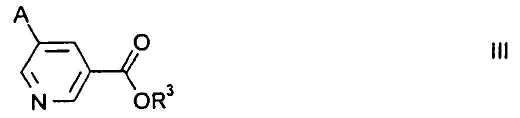 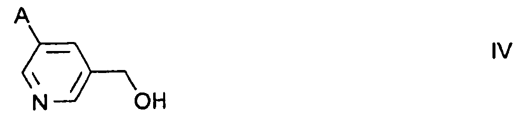 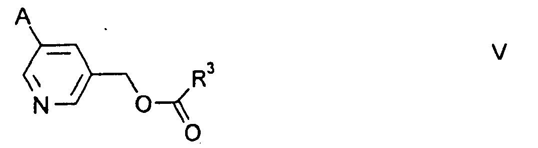 worin A 4-Fluorphenyl bedeutet und R³ Methyl, Ethyl, n-Propyl, iso-Propyl, n-Butyl, sek-Butyl oder tert-Butyl bedeutet,
die Ester der Formeln III und V anschließend nach üblichen Verfahren hydrolysiert, um die freie Säure der Formel II oder den Alkohol der Formel IV zu erhalten und den Alkohol der Formel IV durch übliche Methoden zur Säure Formel II oxidiert.

Die auf diese Weise erhaltene Säure der Formel II kann ohne Weiteres für das erfindungsgemäße Verfahren verwendet werden.

R³ ist bevorzugt Methyl oder Ethyl, insbesondere Methyl.

5-(4-Fluorphenyl)nicotinaldehyd und die neue Verbindung der Formeln II sind ebenfalls Gegenstand der Erfindung.

Die Reaktionsdurchführung des erfindungsgemäßen Verfahrens zur Herstellung von 5-(4-Fluorphenyl)nicotinaldehyd ist einfach, wobei die betreffenden 5-Arylnicotinsäure vorzugsweise in organischen Lösungsmitteln, bei Temperaturen von 10 bis 180°C, vorzugsweise bei 20 bis 150°C und ganz besonders bevorzugt bei 40 bis 100°C und bei einem Druck von 1 bis 150 bar, vorzugsweise bei 1,5 bis 120 bar und insbesondere bei 2 bis 100 bar unter Zusatz eines Carbonsäureanhydrids in Gegenwart eines Palladium-Ligand-Komplexes hydriert werden.

Als Lösungsmittel für das erfindungsgemäße Verfahren werden vorzugsweise Ether, wie z.B. Diethylether oder Tetrahydrofuran, Ketone, wie z.B. Aceton, Kohlenwasserstoffe, wie z.B. Toluol, Benzol, Hexan oder Heptan, Amide wie z.B. Dimethylformamid, N-Methylpyrrolidon, oder Säureanhydride, wie z.B. Acetanhydrid verwendet werden.
Besonders bevorzugt sind Ether, insbesondere Tetrahydrofuran.

Mischungen der genannten Lösungsmittel können ebenfalls Verwendung finden.

Die Menge des Lösungsmittels ist nicht kritisch, vorzugsweise können 10g bis 500 g Lösungsmittel je g der umzusetzenden Verbindung der Formel II zugesetzt werden.

Die Dauer der Umsetzung hängt von den gewählten Reaktionsbedingungen ab. In der Regel beträgt die Reaktionsdauer 0.5 Stunden bis 10 Tage, vorzugsweise 1 bis 24 Stunden.

In einer bevorzugten Ausführungsform der Erfindung wird das Ende der Reduktion zum Aldehyd durch geeignete Analysemethoden, z.B. HPLC, ermittelt und die Reduktion unterbrochen.

Als Hydrier-Katalysator wird ein Palladium-Ligand-Komplex verwendet.

Als Liganden werden Phosphane, vorzugsweise mit drei organischen Resten, insbesondere Alkyl- und Arylresten verwendet. Generell können als Liganden einzähnige oder zweizähnige Phosphane eingesetzt werden. Geeignete einzähnige Phosphande sind beispielsweise (CH₃)₃P, (C₂H₅)₃P, (C₃H₇)₃P, (C₄H₉)₃P, (C₅H₁₁)₃P, (C₆H₁₃)₃P, (C₇H₁₅)₃P, (C₈H₁₇)₃P, (C₆H₁₁)₃P, (C₆H₅)₃P, (CH₃C₆H₄)₃P, (C₆H₅CH₂)₃P, (C₄H₉)₂(C₆H₅)P, (C₄H₉) (C₆H₅)₂P.Die obigen Summenformeln schließen die verzweigten und cyclischen Kohlenwasserstoffreste ein. Bevorzugt sind n-Propyl, n-Butyl, sek.-Butyl, tert.-Butyl sowie Cyclohexyl als Kohlenwasserstoffreste. Phosphane mit identischen Resten sind bevorzugt.

Geeignete zweizähnige Liganden sind beispielsweise: (C₆H₅)₂PCH₂(CH₂)_{q}(C₆H₅)₂, 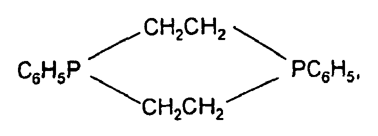 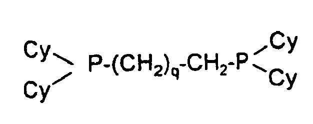 worin Cy Cyclohexyl und q 1, 2 oder 3, insbesondere 1 oder 3 bedeutet.

Als Liganden werden bevorzugt eingesetzt: Triphenylphosphan, Diphenylphophinoferrocen, Tri-o-tolylphosphin, 1,2-Bis(diphenylphosphino)ethan, 2,4-Bis(diphenylphosphino)pentan, Bis(diphenylphosphino)methan, Tris-(tert-butyl)phosphan. Es ist ebenfalls möglich Mischungen der Liganden zu verwenden.

Palladiumverbindungen können der Oxidationsstufe II, d.h. als Salz oder in der Oxidationsstufe 0 eingesetzt werden. Bevorzugt wird es in der Oxidationsstufe 0, vorzugsweise durch Phosphane komplexiert, eingesetzt.

Palladium wird in der Regel als Palladiumverbindung eingesetzt, aus der durch Ligandenzusatz der entsprechende Katalysator hergestellt wird. Es ist ebenfalls möglich, Palladium als stöchiometrisch korrekt zusammengesetzten Komplex einzusetzen, der gemäß der Erfindung mit einer Menge an Ligand versetzt wird, so daß der erfindungsgemäß definierte Ligandenüberschuß eingehalten wird.

Als Palladiumverbindungen, die in Gegenwart des Ligandenüberschusses eingesetzt werden, kommen bevorzugt in Frage:
Tetrakis(triphenylphosphan)palladium(0), Palladium(0)dibenzyliden-Komplexe, Palladium auf Kohle (vorzugsweise 5%ig), PdCl₂ppf, Palladiumacetat-(tri-0-tolylphosphan)-Komplex, Pd(0)*dppe. Pd(0)*dppp. Pd(0)*dppm, Pd(COD)Cl₂, PdCl₂, Pd(OAc)₂, PdBr₂.

insbesondere bevorzugt sind Tetrakis(triphenylphosphan)palladium(0), Palladium(0)dibenzyliden-Komplexe, Palladium auf Kohle (vorzugsweise 5%ig), PdCl₂dppf, Palladiumacetat-(tri-0-tolylphosphan)-Komplex, Pd(0)*dppe, Pd(0)*dppp sowie Pd(0)*dppm.

Das molare Verhältnis von Palladium zur zu hydrienden Säure beträgt vorzugsweise 1 zu 500 bis 1 zu 200, insbesondere 1 zu 50 bis 1 zu 100.

Das molare Verhältnis von Palladium zu Ligand bei einzähnigen Liganden bei 1 zu 5 bis 1 zu 15 liegt vorzugsweise bei 1 zu 5 bis 1 zu 10, insbesondere 1 zu 5 bis 1 zu 6, und bei zweizähnigen Liganden bei 1 zu 2,5 bis 1 zu 7,5 vorzugsweise bei 1 zu 2,5 bis 1 zu 5, insbesondere 1 zu 2,5 bis 1 zu 3.

Als Anhydride die nicht als Lösungsmittel verwendet werden, werden für das Verfahren vorzugsweise Pivalinsäureanhydrid, Esssigsäureanhydrid, Isobuttersäureanhydrid, 5-Norbornen-2,3-dicabonsäureanhydrid, 1,2,3,6-Tetrahydrophthalsäureanhydrid und Naphthalin-1,8-dicarbonsäureanhydrid.

Das molare Verhältnis von Carbonsäureanhydrid zur 5-Arylnicotinsäure betragt vorzugsweise 10 zu 1 bis 8 zu 1, bevorzugt 7 zu 1 bis 6 zu 1 und insbesondere 5 zu 1 bis 2 zu 1.

Durch übliche Aufarbeitungschritte wie z.B. Wasserzugabe zum Reaktionsgemisch und Extraktion können die Verbindungen der Formel I nach Entfernung des Lösungsmittels erhalten werden. Es kann vorteilhaft sein, zur weiteren Reinigung des Produktes eine Destillation oder Kristallisation anzuschließen.

Der nach dem erfindungsgemäßen Verfahren herstellbare 5-(4-Fluorphenyl)nicotinaldehyd stellt ein bedeutendes Zwischen- bzw. Endprodukt in der organischen industriellen Chemie dar. Entsprechend substituierte Derivate stellen insbesondere wertvolle Zwischenprodukte zur Synthese hochveredelter Endprodukte dar, bzw. sind selbst solche Endprodukte, für den Pflanzenschutz, wie z.B. Fungizide, Insektizide, Herbizide oder Pestizide oder zur Herstellung von pharmazeutisch hochwirksamen Substanzen.

Auch ohne weitere Ausführungsformen wird davon ausgegangen, daß ein Fachmann die obige Beschreibung im weitesten Umfang nutzen kann. Die bevorzugten Ausführungsformen sind deswegen lediglich als beschreibende, keineswegs als in irgendeiner Weise limitierende Offenbarung aufzufassen.

Das folgende Beispiel soll die Erfindung erläutern, ohne sie zu begrenzen. Sofern nichts anderes angegeben ist, bedeuten Prozentangaben Gewichtsprozent. Alle Temperaturen sind in Grad Celsius angegeben.

### Beispiel 1:

13.2g 4-Fluorbenzolboronsäure und 10.8g Natriumhydrogencarbonat werden vorgelegt und unter Rühren und Schutzgas mit einer Mischung aus 16.1g 5-Brom-3-hydroxymethyl-pyridin, 30ml Wasser und 60ml Toluol versetzt. Anschliessend werden weitere 14ml Wasser, 28ml Toluol und 0,5g Tetrakis-triphenylphosphin-palladium(0) zugegeben. Das Reaktionsgemisch wird zum Rückfluss erhitzt und 2 Stunden bei dieser Temperatur gehalten. Nach Abkühlen wird wie üblich aufgearbeitet wodurch 5-(4-Fluorphenyl)-nicotinalkohol erhalten wird. Anschließend wird der Alkohol nach üblichen Methoden zur 5-(4-Fluorphenyl)-nicotinsäure oxidiert.

### Beispiel 2:

1,38 kg 5-Bromnicotinsäure, 1,10 kg 4-Fluorbenzolboronsäure und 0,40 kg Pd-Aktivkohle (5%ig) werden in 8,5 Liter Wasser vorgelegt. Die schwarze Suspension wird mit 1,39 kg 32%iger Natronlauge alkalisch gestellt und auf 100 °C erwärmt. Nach 4 Stunden wird wie üblich aufgearbeitet, wodurch 5-(4-Fluorphenyl)-nicotinsäure erhalten wird
Schmelzbereich: 198,5 °C bis 203 °C.

### Beispiel 3:

15,3 g 5-(4-Fluorphenyl)-nicotinsäuremethylester (erhältlich durch Suzuki-Kupplung von 5-Brompyridin-3-carbonsäuremethylester mit 4-Fluorbenzolboronsäure analog zu Beispielen 1 oder 2) werden nach üblichen Methoden zur 5-(4-Fluorphenyl)-nicotinsäure verseift.

### Beispiel 4:

5-Brom-3-Acetoxymethyl-pyridin wird gemäß Beispiel 1 mit 4-Fluorbenzolboronsäure umgesetzt. Der erhaltene Essigsäure-5-(4-fluorphenyl)-pyridin-3-ylmethylester wird nach üblichen Methoden verseift und anschließend zur 5-(4-Fluorphenyl)-nicotinsäure oxidiert.

### Beispiel 5:

5-(4-Fluorphenyl)-nicotinsäure wird im Autoklaven in Tetrahydrofuran gelöst, mit drei Äquivalenten des jeweiligen Säureanhydrids versetzt und die Mischung durch mehrmaliges Aufpressen von Schutzgas inertisiert. Eine Lösung von Pd(OAc)₂ und PPh₃ in Tetrahydrofuran wird im Schutzgasgegenstrom zugegeben und die Reaktionslösung anschließend unter einem Wasserstoffdruck von 8 bar bei 80°C unter Rühren hydriert. Durch übliche Aufarbeitung wird der Aldehyd in einer Ausbeute von 98 % erhalten.

## Patentansprüche

1. Verfahren zur Herstellung von 5-(4-Fluorophenyl)nicotinaldehyd durch Reduktion von 5-(4-Fluorophenyl)nicotinsäure mittels katalytischer Hydrierung in Gegenwart von Carbonsäureanhydriden, bei der als Katalysator ein Palladium-LigandKomplex verwendet wird, **dadurch gekennzeichnet, daß** das molare Verhältnis von Palladium zu Ligand bei einzähnigen Liganden bei 1 zu 5 bis 1 zu 15 und bei zweizähnigen Liganden bei 1 zu 2,5 bis 1 zu 7,5 liegt.

2. 5-(4-Fluorophenyl)nicotinaldehyd.

3. 5-(4-Fluorophenyl)nicotinsäure.

4. Verwendung von 5-(4-Fluorophenyl)nicotinaldehyd zur Herstellung von (R)-2 [5-(4-Fluorophenyl)-3-pyridylmethylaminomethyl]-chroman,
**dadurch gekennzeichnet, dass**
(a) 5-(4-Fluorophenyl)nicotinaldehyd nach dem Verfahren gemaess Anspruch 1 hergestellt wird,
(b) der so hergestellte 5-(4-Fluorophenyl)nicotinaldehyd mit R-Chromanamin umgesetzt wird und
(c) das dabei erhaltene Imin reduziert wird.

## Claims

1. Process for the preparation of 5-(4-fluorophenyl)nicotinaldehyde by reduction of 5-(4-fluorophenyl)nicotinic acid by catalytic hydrogenation in the presence of carboxylic anhydrides, in which the catalyst used is a palladium/ligand complex, **characterised in that** the palladium to ligand molar ratio is 1:5 to 1:15 in the case of monodentate ligands and 1:2.5 to 1:7.5 in the case of bidentate ligands.

2. 5-(4-Fluorophenyl)nicotinaldehyde.

3. 5-(4-Fluorophenyl)nicotinic acid.

4. Use of 5-(4-fluorophenyl)nicotinaldehyde for the preparation of (R)-2-[5-(4-fluorophenyl)-3-pyridylmethylaminomethyl]chroman,
**characterised in that**
(a) 5-(4-fluorophenyl)nicotinaldehyde is prepared by the process according to Claim 1,
(b) the 5-(4-fluorophenyl)nicotinaldehyde prepared in this way is reacted with R-chromanamine and
(c) the resultant imine is reduced.

## Revendications

1. Procédé de préparation de 5-(4-fluorophényl)nicotinaldéhyde par réduction de l'acide 5-(4-fluorophényl)nicotinique par hydrogénation catalytique en présence d'anhydrides carboxyliques, dans lequel le catalyseur utilisé est un complexe de palladium/ligand, **caractérisé en ce que** le rapport molaire du palladium au ligand va de 1:5 à 1:15 dans le cas des ligands monodentés et de 1:2,5 à 1:7,5 dans le cas des ligands bidentés.

2. 5-(4-Fluorophényl)nicotinaldéhyde.

3. Acide 5-(4-fluorophényl)nicotinique.

4. Utilisation de 5-(4-fluorophényl)nicotinaldéhyde pour la préparation de (R)-2-[5-(4-fluorophényl)-3-pyridylméthylaminométhyl]chromane,
**caractérisée en ce que**
(a) le 5-(4-fluorophényl)nicotinaldéhyde est préparé par le procédé selon la revendication 1,
(b) le 5-(4-fluorophényl)nicotinaldéhyde ainsi préparé est réagi avec de la R-chromanamine et
(c) l'imine résultante est réduite.
